# EUROPEAN PATENT SPECIFICATION

(11) **EP 4 151 744 B1**
(45) Date of publication and mention of the grant of the patent: **20.11.2024**
(21) Application number: 20935377.0
(22) Date of filing: 12.05.2020
(51) Int. Cl.: C12Q 1/06, C12Q 1/18

(54) **AUTOMATIC ANALYZER AND AUTOMATIC ANALYSIS METHOD**
AUTOMATISCHER ANALYSATOR UND AUTOMATISCHES ANALYSEVERFAHREN
ANALYSEUR AUTOMATISÉ ET PROCÉDÉ D'ANALYSE AUTOMATIQUE

(43) Date of publication of application: 22.03.2023
(73) Proprietor: HITACHI HIGH-TECH CORPORATION, Tokyo 105-6409 (JP)
(72) Inventor: SUGIYAMA Kiyotaka, Tokyo 100-8280 (JP); FUJITA Hiroko, Tokyo 105-6409 (JP)
(74) Representative: Strehl Schübel-Hopf & Partner
(86) International application number: PCT/JP2020/018899
(87) International publication number: WO 2021/229667

(56) References cited:
- EP-B1- 2 364 447
- WO-A1-2019/097752
- JP-A- 2005 503 803
- JP-A- 2007 006 709
- JP-A- 2007 006 709
- JP-A- 2013 512 685
- JP-A- 2014 235 076
- JP-A- H 078 292
- US-A1- 2004 185 437
- BROYER PATRICK ET AL: "An Automated Sample Preparation Instrument to Accelerate Positive Blood Cultures Microbial Identification by MALDI-TOF Mass Spectrometry (Vitek?MS)", FRONTIERS IN MICROBIOLOGY, vol. 9, 15 May 2018 (2018-05-15), XP093104157, DOI: 10.3389/fmicb.2018.00911

## Description

### Technical Field

The present invention relates to an automatic analyzer that analyzes a sample containing bacteria and blood cells.

### Background Art

Sepsis is a highly lethal infection, and it is important to promptly perform diagnosis and appropriate treatment based thereon. When sepsis is determined, a blood culture test is usually performed. This is to determine whether bacteria are present in blood that is a sterile sample. Generally, a smear test is then performed, and then an identification test and a sensitivity test are performed. In the identification test, a blood culture positive sample is isolated and cultured, and the type of bacteria is specified for the obtained colony. The sensitivity test measures the sensitivity of the bacteria to antimicrobials. The series of tests described above requires one day for the blood culture test, one day for the isolation and cultivation, and one day for the sensitivity test, and thus requires a total test time of two to three days. That is, it currently takes two to three days to determine whether treatment with an appropriate antimicrobial has been performed. Therefore, if an ineffective antimicrobial has been administered so far, the lethality of sepsis is extremely high.

In the blood culture test, very few bacteria, on the order of about 10 CFU/mL (CFU: colony forming unit) included in the case of sepsis, are grown in a culture bottle. In general, culture is performed for about 8 hours to overnight, and bacteria are grown until a gas component or the like generated by respiration, fermentation or the like of bacteria reaches a detectable level. It is known that a culture bottle positive for blood culture contains 10⁶ to 10¹⁰ CFU/mL of bacteria. Since the concentration of bacteria at the time of blood culture positive varies depending on the condition of the blood of a patient, bacterial species, a blood culture test device and the like, the concentration of bacteria is in such a wide range. Main components other than bacteria in the blood culture bottle include a resin, beads, activated carbon and the like that adsorb antibiotics, in addition to blood cell components and a medium. Among them, the concentration of red blood cells and white blood cells present in blood is high, about 10⁹ cells/mL and 10⁷ cells/mL, respectively, and is equal to or higher than the concentration of bacteria.

In the isolation and cultivation, a sample positive for blood culture is applied to an agar medium to grow colonies. By predicting the type of bacteria from the properties of colonies and the like and more reliably identifying the type of bacteria, and by preparing a bacterial suspension from colonies, it is possible to obtain a sample having no impurities other than bacteria and having a bacterial concentration (generally 10⁵ to 10⁶ CFU/mL) necessary for sensitivity test.

In the sensitivity test, generally, a certain concentration of an antimicrobial is introduced into a bacterial suspension containing bacteria, and the degree of growth of the bacteria is determined according to the concentration of the antimicrobial. Since the result of the sensitivity test varies, it is important to adjust in advance the bacterial concentration in the bacterial suspension to be constant. Regarding sensitivity test, studies to speed up the time until the end of test are currently in progress. The current golden standard method measures the degree of growth of bacteria by a change in turbidity, and it takes a whole day for the test. Currently, a method of more rapidly determining turbidity change using laser light, a method of rapidly determining the degree of growth of individual bacteria by a microscope, a method of rapidly quantifying the degree of growth of bacteria by ATP (adenosine triphosphate) light emission and the like are being developed, and the time required for sensitivity test may be shortened to about several hours. On the other hand, as a pretreatment step for preparing a bacterial suspension, a method of performing isolation and cultivation for one day and diluting colonies in a liquid is still used.

In contrast, if a bacterial suspension with a certain concentration of 10⁵ to 10⁶ CFU/mL can be prepared in a short time by removing components other than bacteria (for example, blood cell components, impurities contained in the culture medium, and the like) from the blood culture-positive sample without performing the isolation and cultivation, the time required for the sensitivity test is further shortened by one day.

In response to such a problem, PTL 1 discloses a method of selectively destroying only blood cell components without affecting growth of bacteria by using two different surfactants. PTL 2 discloses a method of decomposing a blood cell by a protease, dilating the blood cell with a hypotonic solution, and selectively destroying only a blood cell component using a surfactant.

PTL 3 discloses a method of fluorescently labeling bacteria captured on a membrane filter and detecting the number and concentration of bacteria. According to this method, even when impurities other than bacteria are contained, the concentration of bacteria can be measured.

### Citation List

### Patent Literature

PTL 1: JP 2014-235076 A
PTL 2: WO 2019/097752
PTL 3: JP 2007-006709 A

Other conventional analysis methods in this field are described in US 2004/185437 A1, EP 2 364 447 B1, and by Broyer et al. in "An Automated Sample Preparation Instrument to Accelerate Positive Blood Cultures Microbial Identification by MALDI-TOF Mass Spectrometry (Vitek®MS)", in Frontiers in Microbiology, Vol. 9, 15 May 2018.

### Summary of Invention

### Technical Problem

However, PTLs 1 to 2 do not disclose a means for adjusting the concentration of bacteria to be constant. For example, in general, when a bacterial suspension is prepared from colonies, it is possible to adjust the concentration of bacterial suspension based on the value of turbidity. However, since an absorption wavelength of a blood cell component such as a red blood cell, a white blood cell or a platelet, hemoglobin contained in a large amount in the blood cell or the like is the same as a wavelength band used for measuring scattered light of bacteria, adjustment by turbidity is difficult.

In PTL 3, it is necessary to treat bacteria with a staining reagent for fluorescent labeling, and there is a possibility that the properties of bacteria are changed by being exposed to the reagent during the treatment, and the result of the sensitivity test is affected. Therefore, the method requiring such a staining step is difficult to apply for sensitivity test. In addition, there is also a problem that reagent cost is high and a dedicated expensive optical system by fluorescence excitation is required.

The present invention has been made in view of such a situation, and provides a technique of estimating a bacterial concentration in a sample comprising bacteria and blood cells.

### Solution to Problem

An automatic analyzer according to the present invention introduces a substance that destroys blood cells in sample in which bacteria and the blood cells are mixed, separates the destroyed blood cells and the bacteria, and then takes out the bacteria by a filter, and estimates the concentration of bacteria in the sample according to correspondence data between the amount of blood cells remaining on the filter and the concentration of bacteria in the sample.

### Advantageous Effects of Invention

According to the automatic analyzer of the present invention, the bacterial concentration in the sample can be estimated from the sample in which bacteria and blood cells are mixed. As a result, the sensitivity test can be accurately performed. The present invention is defined by the independent claims. The dependent claims depict additional embodiments of the invention.

### Brief Description of Drawings

[FIG. 1] FIG. 1 is a flowchart showing a general procedure for destroying blood cells from a blood sample containing bacteria to remove blood cells.
[FIG. 2] FIG. 2 is an example of an image obtained by imaging a filtration filter without staining.
[FIG. 3] FIG. 3 is a graph showing a relationship between color information of a filter calculated by processing a filter image and the number of red blood cells in a sample passed through the filter.
[FIG. 4] FIG. 4 is a graph showing a relationship between color information of a filter calculated by processing a filter image and an actual blood bacterial concentration.
[FIG. 5] FIG. 5 is a flowchart showing a procedure of performing bacterial concentration adjustment using a blood bacterial concentration estimated from a filter image.
[FIG. 6] FIG. 6 is a configuration diagram of an automatic analyzer 100 according to Aspect 2.
[FIG. 7] FIG. 7 shows results of bacterial concentration adjusted from a blood culture-positive sample by turbidity measurement.
[FIG. 8] FIG. 8 shows results of bacterial concentration adjusted from a blood culture-positive sample using the method shown in FIG. 5.
[FIG. 9] FIG. 9 shows growth rates of blood culture-positive samples and a sample created from colonies.
[FIG. 10] FIG. 10 shows growth rates of blood culture-positive samples and a sample created from colonies.
[FIG. 11] FIG. 11 shows results of performing a drug sensitivity test.

### Description of Aspects

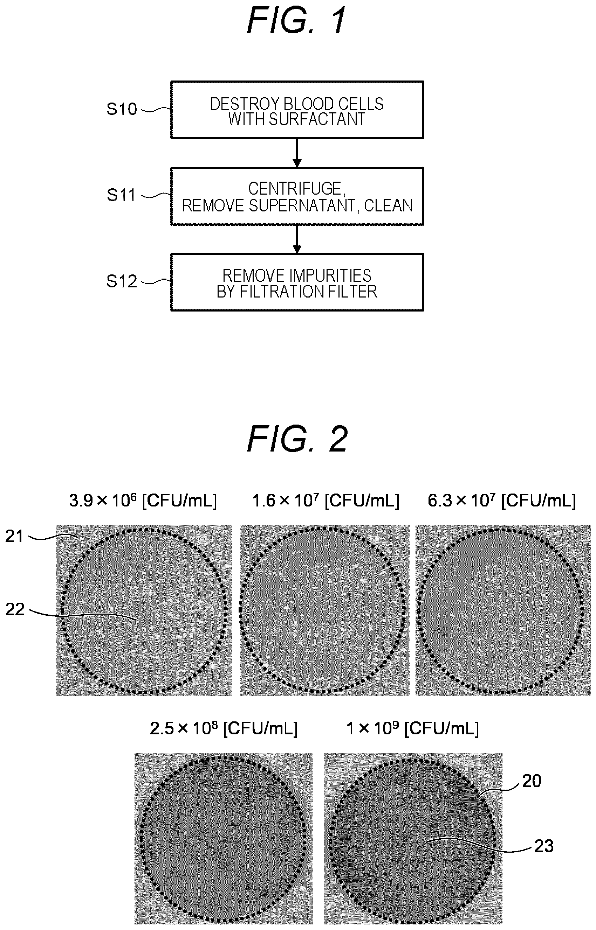

FIG. 1 is a flowchart showing a general procedure for destroying blood cells from a blood sample containing bacteria to remove blood cells. Prior to the aspects of the present disclosure a general procedure for removing blood cells from a blood cell sample will be described with reference to FIG. 1. Thereafter, the aspects of the disclosure will be described in detail.

In step S10, a surfactant is added to the blood sample to destroy blood cells. As the amount of blood to be treated increases, the number of bacteria finally obtained increases, and thus it is preferable to pretreat a larger sample. However, since the amount of waste liquid also increases, it is preferable to pretreat about several mL to 10 mL per sample. The surfactant is preferably (a) an anionic surfactant having hydrophilic and hydrophobic moieties and the hydrophobic moiety being a chain hydrocarbon, or (b) a surfactant having hydrophilic and hydrophobic moieties and the hydrophobic moiety having a cyclic hydrocarbon, or a combination of (a) and (b). Specifically, the former includes sodium dodecyl sulfate, lithium dodecyl sulfate, and sodium N-lauroyl sarcosine, and the latter includes saponin, sodium cholate, sodium deoxycholate, 3-[(3-cholamidopropyl)dimethylammonio]-1 propanesulfonate, and 3-[(3-cholamidopropyl)dimethylammonio]-2-hydroxy-1 propanesulfonate. The next step S11 may be performed immediately after the addition of the surfactant, but the reaction may be allowed to stand for about 5 to 15 minutes to wait for completion of the reaction.

In step S11, centrifugation is performed in order to remove components in the blood cells destroyed by the surfactant and flowed out, for example, hemoglobin and the like, and then, the supernatant is removed and cleaned. In this step, for example, it is preferable to perform centrifugation at 2000 G for about 5 to 10 minutes. However, it is sufficient as long as bacteria and blood cell components that have not been destroyed by the surfactant and hemoglobin and the like that have flowed out can be separated, and the centrifugation speed and the centrifugation time are not limited thereto. The cleaning is performed using pure water, physiological saline or the like, and may be performed only once or a plurality of times.

In step S12, the sample is filtered by a filter in order to further remove the blood cell component that could not be destroyed by the surfactant and impurities in the medium. By using a filter with a pore size (mesh interval) larger than that of bacteria, bacteria are allowed to pass through, and impurities other than bacteria are captured by the filter. For example, it is preferable to use a filter with a pore size of 1 to 40 µm. When the amount of impurities is large, filtration may be performed a plurality of times, such as filtration with a filter with a large filtration pore size and then filtration with a filter with a small filtration pore size. In order to prevent bacteria from being captured in the filter, it is preferable to use a filter made of a hydrophobic material. By the above steps S10 to S12, it is possible to remove impurities other than bacteria from the blood sample and to extract bacteria.

### <Aspect 1>

In aspect 1 of the disclosure, a method is shown that obtains a sample having a bacterial concentration adjusted to a desired value from a blood sample containing bacteria. Note that the present aspect is merely an example, and is not limited to this configuration. Pretreatments of S10 to S12 were performed using blood samples containing E. coli and S. aureus. The blood samples were prepared according to the following procedure. Into a blood culture bottle containing drug-adsorbing beads, 10 mL of blood derived from a healthy volunteer and 0.1 mL of a bacterial suspension whose concentration had been previously adjusted to about 150 CFU/mL from colonies were introduced to prepare blood equivalent to that of an actual sepsis patient. Thereafter, the sample was introduced into a blood culture device and cultured, and when the blood culture became positive, the sample was taken out and used for an experiment. In addition, a sample corresponding to a negative control having a blood bacterial concentration of 0 CFU/mL cultured without introducing the bacterial suspension was also prepared, and the bacterial concentration was changed by appropriately diluting the sample.

FIG. 2 is an example of an image obtained by imaging a filtration filter without staining. Here, the results of treating blood samples created in advance so that the concentration of E. coli in the blood samples is 10⁶ to 10⁹ CFU/mL are shown. A filter region 20 surrounded by a broken line is a region of interest. In a case where the actual blood bacterial concentration is 3.9 × 10⁶ CFU/mL, a region 22 in which impurities do not exist exhibiting the same tone as that of an outer filter region 21 occupies the most part. In a case where the actual blood bacterial concentration is 1 × 10⁹ CFU/mL, an area 23 where impurities exist with strong redness occupies the most part, and the redness becomes stronger as the actual blood bacterial concentration increases.

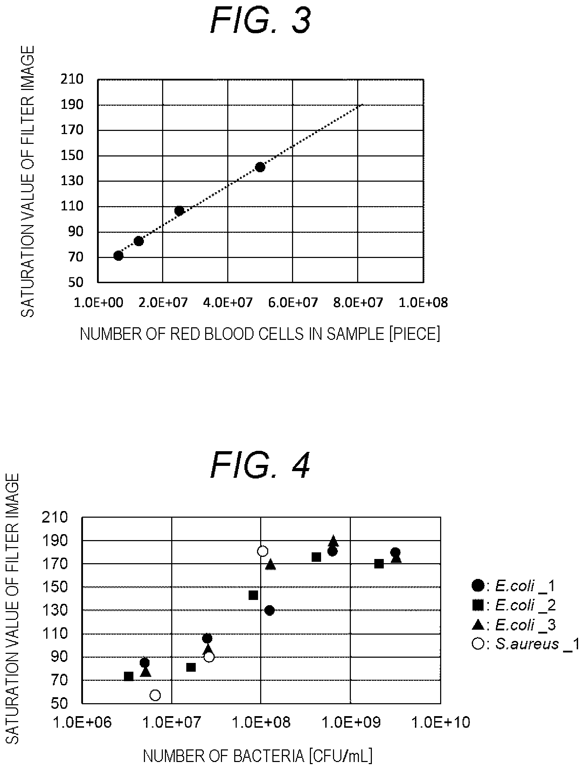

FIG. 3 shows results of filtration using a mixed solution of a negative control blood sample having a blood bacterial concentration of 0 CFU/mL and a surfactant, and the amount of red blood cells in the sample used for filtration and the redness of the filter image were compared. The amount of red blood cells was calculated by a blood cell counter. In order to quantitatively evaluate redness, a saturation value calculated by the following processing was used. The filter image is a color image, and is generally expressed in an RGB color space. RGB was converted into HSV (hue, saturation, and lightness) in order to reduce influence such as ambient brightness at the time of imaging. More specifically, the average value of the saturation values of pixels inside the filter region 20 was calculated as the redness of the filter image. From the results of FIG. 3, it can be seen that there is a positive correlation between the amount of red blood cells in the sample passed through the filter and the saturation value of the filter image. Since the blood sample of the negative control contains not only red blood cells but also platelets and fibrin to which hemoglobin is attached, and impurities in the medium, it is considered that such impurities strengthen the redness of the filter. That is, the amounts of red blood cells and other impurities can be detected from the saturation value of the filter image.

Since the redness of the filter increases according to the amount of impurities such as red blood cells contained in the sample, the reason why the result of FIG. 2 was obtained is estimated as follows. The surfactant is added at a certain concentration regardless of the actual blood bacterial concentration, and most blood cells in the sample are destroyed by the surfactant when the actual blood bacterial concentration is low, so that most blood cells are removed in step S11 and no impurities remain on the filter. On the other hand, when the actual blood bacterial concentration increases, the concentration of bacteria and the concentration of red blood cells become approximately the same to each other, and the bacteria themselves inhibit the action of blood cell destruction by the surfactant. As a result, the amount of red blood cells not completely destroyed in step S11 increases, and the red blood cells not completely destroyed are captured in the filtration step of step S12. In addition, it is also conceivable that bacteria aggregate with hemoglobin, fibrin and platelets, and impurities showing redness are captured in the filtration step of step S12. As the actual blood bacterial concentration increases, the redness of the filter region 20 after filtration becomes stronger. Therefore, for example, by calculating the amount of, for example, red blood cells, which are impurities remaining on the filter after filtration, from the image of the filter, the concentration of bacteria that have passed through the filter can be known.

FIG. 4 is a graph showing a relationship between color information of a filter calculated by processing a filter image and an actual blood bacterial concentration. In order to quantify the color information of the filter, the filter image indicated in the RGB color space was converted into HSV (hue, saturation, and lightness), and the value of saturation was used. Specifically, the average value of the saturation values of pixels inside the filter region 20 is used. FIG. 4 also shows the results of repeated experiments with E. coli and the result with S. aureus. There is a positive correlation between the blood bacterial concentration and the saturation value of the filter image when the actual blood bacterial concentration is in the range of 10⁶ to 10⁹ CFU/mL. When a calibration curve is acquired from the blood bacterial concentration and the saturation value of the filter image, the number of bacteria can be estimated based on the information of the saturation value of the filter image after filtration. This range of the blood bacterial concentration is almost equivalent to the bacterial concentration in a sample that is usually positive in blood culture, and thus can be applied to various bacterial species and strains.

The specific concentration of surfactant used in the treatment can be defined in the following range. Usually, the concentration of red blood cells in blood is on the order of 10⁹/mL, and if a 1 mL sample is pretreated, the number of red blood cells in the sample is 10⁹. Here, the range of impurities that can be estimated from the tone of the filter shown in FIG. 2, for example, the amount of red blood cells is 10⁶ to 10⁸. That is, in order to be able to estimate the actual bacterial concentration from the tone of the filter, the red blood cells may be destroyed until the concentration reaches 1/1000 to 1/10. That is, a surfactant at a concentration capable of destroying 90 to 99.9% of red blood cells in blood containing bacteria is required.

Note that the range of the surfactant concentration shown here is, for example, a case where 1 mL of sample is pretreated, and when the volume to be treated increases, it is preferable to increase the concentration of the surfactant so that the destruction rate of red blood cells increases accordingly. For example, when 10 mL of sample is pretreated, a surfactant having a concentration capable of destroying 99 to 99.99% of red blood cells is required, and the concentration may vary depending on the throughput of the sample.

Specifically, the concentration of the surfactant capable of destroying 99 to 99.99% of red blood cells in blood containing bacteria is in the range of 0.05 wt% to 0.5 wt%, assuming an example of sodium dodecyl sulfate, which is an anionic surfactant having hydrophilic and hydrophobic moieties and the hydrophobic moiety being a chain hydrocarbon. Regarding other surfactants such as lithium dodecylsulfate and sodium N-lauroyl sarcosine, the concentration of the surfactant is preferably a concentration capable of destroying a desired red blood cell.

As other surfactants, for example, regarding saponin, sodium cholate, sodium deoxycholate, 3-[(3-cholamidopropyl)dimethylammonio]-1 propanesulfonate, and 3-[(3-cholamidopropyl)dimethylammonio]-2 hydroxy-1 propanesulfonate, which are surfactants having hydrophilic and hydrophobic moieties and a cyclic hydrocarbon as a hydrophobic moiety, the types of surfactants may be mixed.

In Aspect 1, 2.7 mL of blood sample was treated with a surfactant such as sodium dodecyl sulfate having a final concentration in the range of 0.05 wt% to 0.5 wt%, capable of destroying 99 to 99.99% of red blood cells in blood containing bacteria.

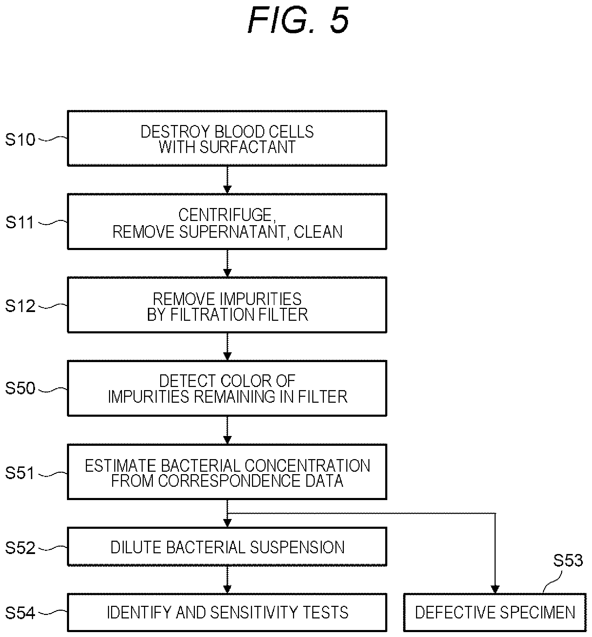

FIG. 5 is a flowchart showing a procedure of performing bacterial concentration adjustment using a blood bacterial concentration estimated from a filter image. Steps S10 to S12 are the same as the steps shown in FIG. 1.

In step S50, the filter is imaged, and the color of the impurities remaining on the filter is acquired. In this flowchart, since the amount of impurities is estimated based on the color of red blood cells remaining on the filter, it is not necessary to stain a sample and impurities.

In step S51, correspondence data describing correspondence between the color of the impurities remaining on the filter and the actual blood bacterial concentration is read out, and the estimated blood bacterial concentration is calculated by referring to the correspondence data using the color acquired from the filter image. Specifically, regarding the color of the impurities remaining on the filter and the actual blood bacterial concentration, for example, a calibration curve represented by a single logarithm is acquired, and the blood bacterial concentration estimated from the data of the calibration curve is calculated. The correspondence data is illustrated in FIG. 4, and is created in advance and stored in the storage device. Note that the method of the present disclosure can be applied as long as there is at least one piece of correspondence data regardless of the bacterial species and the type of surfactant. In the case of obtaining the blood bacterial concentration estimated more accurately, the correspondence data may be held for each of bacterial species, or may be held depending on information of resistant strains such as methicillin-resistant Staphylococcus aureus, and the type of surfactant.

In step S52, the sample is diluted to obtain a desired bacterial concentration based on the blood bacterial concentration estimated in S51. For example, when the blood bacterial concentration estimated in S51 is 5 × 10⁸ CFU/mL, if the desired bacterial concentration is 5 × 10⁵ CFU/mL, 1000-fold dilution is performed. When the blood bacterial concentration estimated in S51 has not reached the desired bacterial concentration, it is preferable to proceed to step S53 and determine that the specimen is a defective specimen. When it is determined as a defective specimen, it is difficult to prepare a specimen suitable for sensitivity test, and thus the blood culture bottle is further cultured to grow the bacteria, and then the process returns to step S10. Alternatively, an identification test or a sensitivity test may be performed using colonies obtained by performing isolation and cultivation.

In step S54, an identification test and a sensitivity test are performed using the prepared bacterial suspension. Any method may be used as the inspection method. Examples thereof include such as an identification test using an automatic device, a genetic test, a sensitivity test by a microliquid dilution method, a sensitivity test by a disk method, and a rapid sensitivity test by a microscopic image, laser scattered light measurement or the like.

### <Aspect 1: Summary>

In Aspect 1, the sample after destroying the blood cells is filtered by the filtration filter, and the estimated blood bacterial concentration is calculated by referring to the correspondence data between the color component of the image of the impurities remaining on the filter and the actual blood bacterial concentration. This makes it possible to create a sample having a desired bacterial concentration without performing isolation and cultivation. Therefore, the isolation and cultivation step that usually requires about one whole day can be shortened to, for example, about 30 minutes.

### <Aspect 2>

In Aspect 2, an automatic analyzer for obtaining a sample having a bacterial concentration adjusted to a desired value from a blood sample containing bacteria is shown. Note that the present aspect is merely an example, and is not limited to this configuration.

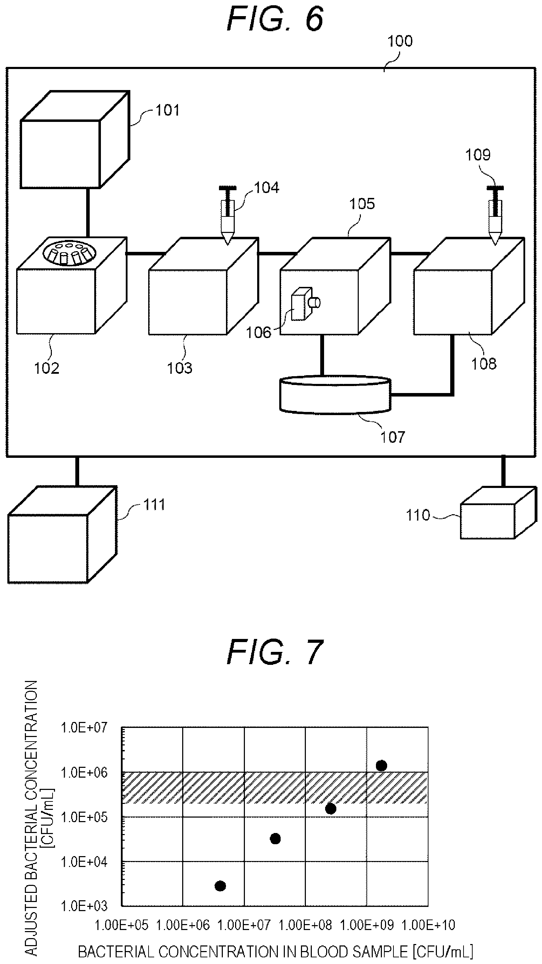

FIG. 6 is a configuration diagram of an automatic analyzer 100 according to Aspect 2. The automatic analyzer 100 is a device that automatically performs the pretreatment procedure described in FIG. 5. Usually, a rubber stopper is used for the blood culture bottle to prevent contamination or the like, and the inside of the bottle is vacuum. An operator takes out a blood sample from the blood culture bottle using an injection needle, and dispenses the blood sample into a container containing a surfactant. Accordingly, S10 is performed. Instead of an operator introducing a surfactant, an introduction device 101 that automatically introduces a surfactant into a sample can be provided as a part of the automatic analyzer 100.

The sample into which the surfactant is introduced is introduced into a centrifugal separator 102. The blood cells in the sample are destroyed by the surfactant. The centrifugal separator 102 separates the eluted hemoglobin and the like from bacteria, blood cells that could not be destroyed, and the like. As a result, the separation step of S11 is performed.

The sample that has been centrifuged is introduced into a cleaning unit 103. The cleaning unit 103 removes the supernatant of the sample, and cleans the sample with, for example, about 1 mL of physiological saline or pure water, or a cleaning liquid such as a medium. A cleaning pipette 104 aspirates the supernatant. A portion from the bottom of the sample container to a certain reference height may be treated as the supernatant. As a result, the rest of S11 is performed. More strictly, it is preferable to provide a liquid position sensor or the like, detect an interface between a portion where bacteria and blood cells are coagulated into a pellet form and a liquid portion, and treat a portion up to the vicinity of the interface position as the supernatant.

The cleaned sample is introduced into a filtration filter unit 105. The filtration filter unit 105 consists of, for example, a disposal filtration filter, a syringe for capturing impurities in the filter, and the like. S12 is performed by the filtration filter unit 105. In some cases, the sample may be filtered using the centrifugal separator 102.

A camera 106 (corresponding to a sensor that detects the amount of impurities) images impurities remaining on the filtration filter unit 105. A storage unit 107 stores the correspondence data described in FIG. 4. A computer 110 (operation unit) converts the RGB image captured by the camera 106 into an HSV color space image, detects a color component (for example, a saturation value) of the impurity region (S50), and calculates the estimated blood bacterial concentration by referring to the correspondence data using the color (S51).

The filtered sample is introduced into a dilution unit 108. The dilution unit 108 adjusts the dilution ratio so as to obtain a desired bacterial concentration on the basis of the blood bacterial concentration estimated by the computer 110. A dilution pipette 109 introduces the diluent according to its dilution ratio. Accordingly, S52 is performed.

The computer 110 automatically performs the above steps by controlling each unit included in the automatic analyzer 100. It is preferred that the computer 110 includes an input/output device and the operator can instruct the computer 110 about the type of bacteria, the desired bacterial concentration, and the like. The computer 110 can change the correspondence data to be referred according to the input bacteria type, and can also change the dilution ratio by the dilution unit 108 according to the desired bacterial concentration. The blood bacterial concentration value estimated by the computer 110 may be output to an output device such as a display, and when the blood bacterial concentration value is equal to or less than the desired bacterial concentration, a flag indicating a defective specimen may be displayed (S53).

The automatic analyzer 100 may include a sensitivity test device 111 in addition to the above configuration. The computer 110 automatically performs the sensitivity test by controlling the sensitivity test device 111. As a result, all processes from S10 to S54 can be automatically performed. As the contents of the sensitivity test, in addition to those described in Aspect 1, the minimum inhibitory concentration and the like described in Examples described later can also be measured.

### <Aspect 3>

It is considered that the method for adjusting the blood bacterial concentration in the blood sample described in Aspects 1 to 2 is influenced to some extent by the amount of red blood cells contained in the original blood. The concentration of human red blood cells varies depending on sex and health condition, but is about 3 × 10⁹ to 6 × 10⁹/mL, and the variation is extremely small as compared with a bacterial concentration range of 10⁶ to 10¹⁰/mL. Therefore, it is considered that the influence on the result of the estimated blood bacterial concentration is small. However, in a case where another detection result of the amount of impurities such as the red blood cell concentration or the hematocrit value is obtained in advance by another blood cell analysis or the like, the result of step S51 can be corrected using the value. As a result, the blood bacterial concentration estimated more accurately can be calculated. As the correction procedure, for example, the following ones are conceivable.

(Correction procedure 1) When the blood bacterial concentration estimated by the operator or the computer 110 performing the procedure of FIG. 5 is an abnormal value (deviates from a predetermined allowable range), another detection result is used instead of the amount of impurities estimated using the filter image. That is, the blood bacterial concentration is estimated by referring to the correspondence data using another detection result.

(Correction procedure 2) The operator or the computer 110 performs the procedure of FIG. 5 to estimate the amount of impurities on the filter one or more times, and further obtain another detection result. The final amount of impurities is obtained by averaging the plurality of amounts of impurities excluding abnormal values. The estimated blood bacterial concentration is calculated by referring to the correspondence data using the amount of impurities.

In the correction procedure, the correspondence data is referred using another detection result obtained by measuring the amount of impurities. Therefore, the correspondence data needs to describe correspondence between the amount of impurities and the actual blood bacterial concentration. For example, the saturation value of the filter image and the amount of impurities corresponding thereto can be described together in the correspondence data, or a conversion formula between the saturation value and the amount of impurities can be defined in advance, and another detection result can be converted into the saturation value using the conversion formula. That is, the correspondence data may describe correspondence between a value representing the amount of impurities remaining on the filter in some form and the actual blood bacterial concentration in the blood sample.

In Aspects 1 to 2, the saturation value of the filter image is used to detect red blood cells remaining in the filter. Instead of the camera 106, an optical sensor that detects absorption or reflection of a specific wavelength corresponding to red can also be used. That is, by using an optical sensor capable of detecting at least the largest RGB component of impurities remaining on the filter, information similar to the saturation value of the image captured by the camera 106 can be obtained. In this case, the correspondence data also needs to describe a numerical value measured by the optical sensor instead of the saturation value. Alternatively, a person may visually measure the amount of impurities based on a color sample, and refer to the correspondence data based on the measurement result. Furthermore, the color sample itself may describe the correspondence between the color of the impurities and the blood bacterial concentration.

### <Example 1>

In Example 1 of the present disclosure, the superiority of the pretreatment method according to the present disclosure will be described together with a comparative example. In Example 1, comparison was made between concentration adjustment using turbidity measurement and concentration adjustment according to the present disclosure, respectively adjusting the recommended bacterial concentration range of the sensitivity test. In the case of turbidity measurement, absorbance measurement at a wavelength of 600 nm was used. In the case of concentration adjustment according to the present disclosure, the pretreatment method shown in FIG. 1 was used. The type, concentration and the like of bacteria and surfactants used are the same as those in Aspect 1.

FIG. 7 shows results of adjusting the bacterial concentration from the bacterial suspension obtained by the pretreatment method shown in FIG. 1 by turbidity measurement used in the conventional bacterial test pretreatment. The hatched area is a recommended bacterial concentration range when performing a sensitivity test, which is defined by the Clinical and Laboratory Standards Institute. The range of this hatching is an area of 5 × 10⁵ CFU/mL (± 60%), and adjustment was attempted to obtain a median value of 5 × 10⁵ CFU/mL. Specifically, a bacterial suspension adjusted to a McFarland turbidity of 0.5 corresponding to a bacterial number concentration of 1.5 × 10⁸ CFU/mL was diluted 300 times by McFarland standards.

When the bacterial concentration in the blood sample is 10⁸ CFU/mL or more, it is possible to adjust the bacterial concentration to the vicinity of a desired concentration range, but when the blood bacterial concentration in the blood sample is 10⁶ to 10⁷ CFU/mL, the number of bacteria after the adjustment is reduced by 1 to 2 digits. This is because hemoglobin that could not be removed in steps S11 and S12, fine particles contained in the medium and the like contribute to an increase in scattered light, so that the value of turbidity increases even though the bacterial concentration is low. Therefore, since the bacterial concentration is estimated excessively, it is difficult to adjust the bacterial concentration by using turbidity measurement. This is also apparent from the fact that the plot of FIG. 7 does not fall within the hatched area showing the recommended bacterial concentration range at all.

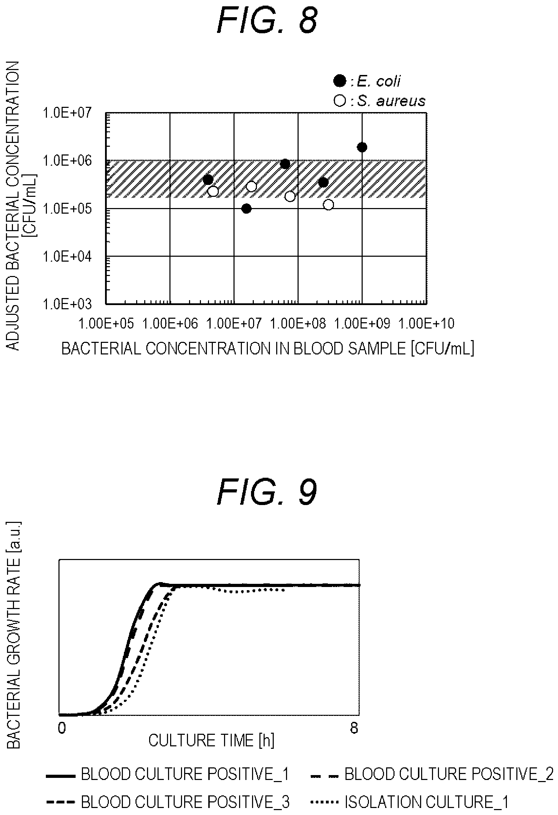

FIG. 8 shows results of performing concentration adjustment from the blood bacterial concentration estimated using the method shown in FIG. 5. As compared with FIG. 7, even when the actual blood bacterial concentration in the blood sample is 10⁶ to 10⁷ CFU/mL, the adjusted bacterial concentration does not decrease and is mostly within the hatched range. Also, FIG. 8 shows results of pretreating E. coli and S. aureus based on the same correspondence data. FIG. 8 shows that even bacteria having greatly different properties such as gram-negative bacteria and gram-positive bacteria can keep the adjusted bacterial concentration within a certain range based on one correspondence data. When it is desired to adjust the concentration more accurately, it is preferable to retain correspondence data for each bacterial species and refer to the correspondence data corresponding to the bacterial species.

### <Example 2>

Example 2 of the present disclosure shows an example in which bacterial concentration was adjusted from a blood culture positive sample by the pretreatment method according to the present disclosure using E. coli. As a comparative example, a growth rate in the case of preparing bacterial suspension from colonies by isolation and cultivation for a whole day is used. The bacterial concentration in a blood culture-positive sample was adjusted to a final bacterial concentration of 5 × 10⁵ CFU/mL using three different bacterial concentrations up to 10⁷ to 10⁹ CFU/mL. Even when bacterial suspension was prepared from colonies, the bacterial suspension was adjusted using turbidity measurement so that the final bacterial concentration was 5 × 10⁵ CFU/mL. 50 µL of the sample was dispensed into a 96-well plate, and 50 µL of Mueller-Hinton medium adjusted to twice the concentration was also dispensed. Thereafter, the 96-well plate was placed in an incubator at 35 to 37°C, and the state of growth was observed with a bright field microscope. Regarding the growth rate, a temporal change in the growth rate was calculated using the area of a region determined to be a bacterium in a microscopic image as an index of the growth rate.

FIG. 9 shows growth rates of blood culture-positive samples and a sample created from colonies. Although there is a difference of about 0.5 hours in the time at which the bacterial growth rate rises between blood culture positive_1 and isolation culture_1, the final growth rates are substantially the same. The same applies to blood culture positive_2 and blood culture positive_3. This indicates that even when the blood culture-positive sample is pretreated, the bacterial concentration can be adjusted to the same degree as the bacterial concentration adjusted from the colonies, and the bacterial growth is not affected in the sensitivity test.

### <Example 3>

In Example 3 of the present disclosure the results when E. coli in Example 2 was changed to S. aureus will be described.

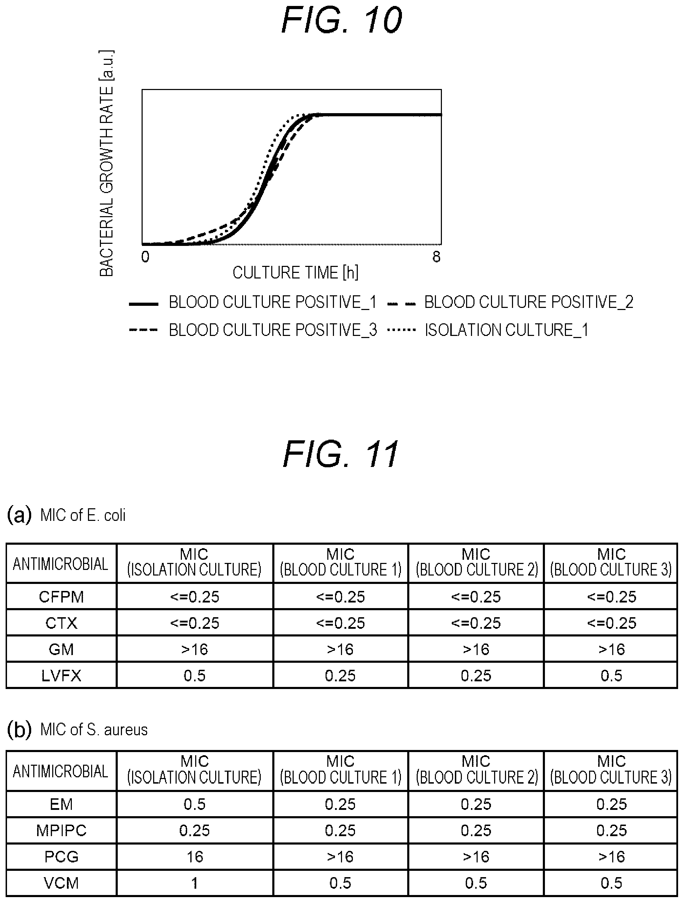

FIG. 10 shows growth rates of blood culture-positive samples and a sample created from colonies. As in Example 2, the final growth rate is the same between blood culture positive and isolation culture. Therefore, as in Example 2, this indicates that even when the blood culture-positive sample is pretreated, the bacterial concentration can be adjusted to the same degree as the bacterial concentration adjusted from the colonies, and the bacterial growth is not affected in the sensitivity test.

### <Example 4>

Example 4 of the present disclosure shows a result of performing a drug sensitivity test on both blood culture positive samples and a sample created from colonies. In the sensitivity test, the lowest concentration (Minimum Inhibitory Concentration: MIC) among the concentrations at which the drug exerts an antibacterial action on bacteria is measured. Here, a microliquid dilution method was used for the sensitivity test. The bacterial suspension and different concentrations of the drug were mixed, and the MIC was determined by visually determining turbidity of each well of the cultured 96-well plate after 18 hours.

FIG. 11 shows results of performing a drug sensitivity test. In FIG. 11, as an example, for E. coli, cefepime (CFPM), cefotaxime (CTX), gentamicin (GM), and levofloxacin (LVFX) were allowed to act, and for S. aureus, erythromycin (EM), oxacillin (MPIPC), penicillin G (PCG), and vancomycin (VCM) were allowed to act.

In any case, the MIC in the case of pretreating the blood culture sample falls within the range of ± 1 tube (twice or half) of the MIC in the case of the sample created from the colonies, indicating that the sensitivity test can be correctly performed even from the sample obtained by pretreating the blood culture sample.

FIG. 11 shows an example in which the MIC is determined using the microliquid dilution method. However, as obtained in FIGS. 8 to 9, the MIC may be determined by a rapid sensitivity test using a microscopic image, or a rapid sensitivity test using a laser beam may be performed.

### <Regarding Modification Example of Present Disclosure>

In the above aspect, it was described that the correspondence data is referred using the average value of the saturation values of the filter region 20, but a maximum value or a mode value may be used instead of the average value. Alternatively, instead of the saturation value, the amount of impurities remaining on the filter may be represented by a feature amount represented by at least two of hue, lightness, and saturation.

In the above aspect, an example in which blood cells contained in the blood sample are detected as impurities has been described, but the present disclosure can also be used for other bacterial samples. That is, the present disclosure can be used for a sample having correspondence between image information obtained by imaging the impurities remaining on the filter and the bacterial concentration in the sample. The substance added to destroy the impurities may be appropriately changed depending on the type of impurities.

### Reference Signs List

- 100: automatic analyzer
- 101: introduction device
- 102: centrifugal separator
- 103: cleaning unit
- 104: cleaning pipette
- 105: filtration filter unit
- 106: camera
- 107: storage unit
- 108: dilution unit
- 109: dilution pipette
- 110: computer

## Claims

1. An automatic analysis method for analyzing a blood sample containing bacteria and impurities, the method comprising:
introducing (S10) a substance that destroys the impurities into the sample;
separating (S11) by a centrifugal separator (102) the impurities and the bacteria from each other in the sample into which the substance has been introduced;
taking out (S12) the bacteria from the sample using a filter (105) that takes out the bacteria from the sample from which the impurities and the bacteria have been separated;
reading correspondence data describing correspondence between a numerical value representing an amount of the impurities remaining on the filter (105) and a concentration of the bacteria in the sample from a storage unit (107) that stores the correspondence data; and
estimating (S51) a concentration of the bacteria in the sample by referring to the correspondence data using a numerical value representing the amount of the impurities remaining on the filter (105) after taking out the bacteria from the sample,
wherein
the sample contains blood cells as the impurities,
the substance is a surfactant,
the surfactant comprises at least one of:
an anionic surfactant having a hydrophilic moiety and a hydrophobic moiety, the hydrophobic moiety being a chain hydrocarbon; or
a surfactant having a hydrophilic moiety and a hydrophobic moiety, the hydrophobic moiety having a cyclic hydrocarbon,
the filter (105) is a filtration filter that separates the impurities and the bacteria by filtering the sample, and
the estimating (S51) the concentration of the bacteria includes
detecting the amount of impurities remaining on the filter (105) using an image obtained by imaging without staining the impurities remaining on the filter (105), and
referring to the correspondence data using the amount of the impurities detected using the image.

2. The automatic analysis method according to claim 1, wherein
the automatic analysis method further comprises capturing (S50) an RGB image as the image, and
the estimating (S51) the concentration of the bacteria includes
converting the RGB image into an HSV color space image, and
using a saturation value on the HSV color space image of the impurities remaining on the filter (105) as the numerical value representing the amount of the impurities remaining on the filter (105).

3. The automatic analysis method according to claim 1, wherein
the automatic analysis method further comprises capturing (S50) an RGB image as the image, and
the estimating (S51) the concentration of the bacteria includes
converting the RGB image into an HSV color space image, and
using a feature amount represented by a hue value, a saturation value, and a brightness value on the HSV color space image of the impurities remaining on the filter (105) as the numerical value representing the amount of the impurities remaining on the filter (105).

4. The automatic analysis method according to claim 1, wherein the estimating (S51) the concentration of the bacteria includes
obtaining a result of detecting the impurities from an optical sensor (106) that detects at least a largest one of RGB color components of the impurities, and
using a result of detection of the impurities remaining on the filter (105) by the optical sensor (106) as the numerical value representing the amount of the impurities remaining on the filter (105).

5. The automatic analysis method according to claim 1, wherein the estimating (S51) the concentration of the bacteria includes
obtaining another detection result by detecting the amount of the impurities in the sample, separately from the amount of the impurities detected in the detecting of the amount of impurities remaining on the filter (105), and
correcting the amount of the impurities detected in the detecting of the amount of the impurities remaining on the filter (105) using the another detection result, and referring to the correspondence data using the corrected amount of the impurities.

6. The automatic analysis method according to claim 1, wherein
the automatic analysis method further comprises performing (S54) a drug sensitivity test of the bacteria to a drug, and
the performing (S54) the drug sensitivity test includes, after the concentration of the bacteria in the sample is estimated, performing the drug sensitivity test for the bacteria in the sample without culturing the bacteria in the sample.

7. The automatic analysis method according to claim 6, wherein
the automatic analysis method further comprises diluting (S52) the sample, and
the diluting (S52) the sample includes, after the concentration of bacteria in the sample is estimated (S51), diluting the sample to create a test sample having a concentration of the bacteria necessary for performing the drug sensitivity test, and
the performing (S54) the drug sensitivity test includes performing the drug sensitivity test on the test sample created in the diluting of the sample.

8. The automatic analysis method according to claim 6, wherein the performing (S54) the drug sensitivity test includes
capturing an image of a sample placed in an incubator maintained at 35 to 37°C by an imaging device (106), and
determining a minimum inhibitory concentration of the drug by measuring a growth rate of the bacteria using an image of the sample captured by the imaging device (106).

9. The automatic analysis method according to claim 1, wherein a filtration pore size of the filter (105) is 1 to 40 µm, and a material of the filter (105) is a hydrophobic material.

10. The automatic analysis method according to claim 1, wherein the impurities include at least red blood cells in blood.

11. An automatic analyzer (100) for analyzing a blood sample containing bacteria and impurities, the automatic analyzer (100) comprising:
a centrifugal separator (102) that is adapted to separate the impurities and the bacteria from each other in the sample into which a substance that destroys the impurities has been introduced;
a filtration filter (105) that is adapted to take out the bacteria from the sample from which the impurities and the bacteria have been separated by filtering the sample;
a storage unit (107) that is adapted to store correspondence data describing correspondence between a numerical value representing an amount of the impurities remaining on the filter (105) and a concentration of the bacteria in the sample; and
an arithmetic unit (110) that is adapted to estimate a concentration of the bacteria in the sample by referring to the correspondence data using a numerical value representing the amount of the impurities remaining on the filter (105) after taking out the bacteria from the sample,
wherein
the sample contains blood cells as the impurities,
the substance is a surfactant,
the surfactant comprises at least one of:
an anionic surfactant having a hydrophilic moiety and a hydrophobic moiety, the hydrophobic moiety being a chain hydrocarbon; or
a surfactant having a hydrophilic moiety and a hydrophobic moiety, the hydrophobic moiety having a cyclic hydrocarbon, and
the arithmetic unit (110) is adapted to estimate the concentration of the bacteria in the sample by
detecting the amount of impurities remaining on the filter (105) using an image obtained by imaging without staining the impurities remaining on the filter (105), and
referring to the correspondence data using the amount of the impurities detected using the image.

## Patentansprüche

1. Automatisches Analyseverfahren zum Analysieren einer Blutprobe, die Bakterien und Verunreinigungen enthält, wobei das Verfahren umfasst:
Einführen (S10) einer Substanz, die die Verunreinigungen in der Probe zerstört;
Trennen (S11) der Verunreinigungen und der Bakterien voneinander in der Probe, in die die Substanz eingebracht wurde, durch einen Zentrifugalseparator (102);
Entnahme (S12) der Bakterien aus der Probe unter Verwendung eines Filters (105), der die Bakterien aus der Probe, aus der die Verunreinigungen und die Bakterien abgetrennt wurden, entnimmt;
Lesen von Korrespondenzdaten, die die Übereinstimmung zwischen einem numerischen Wert, der eine Menge der auf dem Filter (105) verbleibenden Verunreinigungen darstellt, und einer Konzentration der Bakterien in der Probe beschreiben, aus einer Speichereinheit (107), die die Korrespondenzdaten speichert; und
Schätzen (S51) einer Konzentration der Bakterien in der Probe durch Bezugnahme auf die Korrespondenzdaten unter Verwendung eines numerischen Wertes, der die Menge der Verunreinigungen darstellt, die auf dem Filter (105) verbleiben, nachdem die Bakterien aus der Probe entfernt wurden, wobei
die Probe Blutzellen als die Verunreinigungen enthält,
die Substanz ein Tensid ist,
das Tensid mindestens eines der folgenden Elemente umfasst:
ein anionisches Tensid mit einem hydrophilen Teil und einem hydrophoben Teil, wobei der hydrophobe Teil ein Kettenkohlenwasserstoff ist; oder
ein oberflächenaktives Mittel mit einem hydrophilen und einem hydrophoben Teil, wobei der hydrophobe Teil einen zyklischen Kohlenwasserstoff enthält,
der Filter (105) ein Filtrationsfilter ist, der die Verunreinigungen und die Bakterien durch Filtration der Probe abtrennt, und
das Schätzen (S51) der Konzentration der Bakterien umfasst:
Erfassen der Menge der auf dem Filter (105) verbliebenen Verunreinigungen unter Verwendung eines Bildes, das durch Abbilden ohne Färben der auf dem Filter (105) verbliebenen Verunreinigungen erhalten wird, und
Bezugnahme auf die Korrespondenzdaten unter Verwendung der Menge der anhand des Bildes erkannten Verunreinigungen.

2. Automatisches Analyseverfahren nach Anspruch 1, wobei
das automatische Analyseverfahren ferner das Erfassen (S50) eines RGB-Bildes als das Bild umfasst, und
das Schätzen (S51) der Konzentration der Bakterien umfasst
Umwandeln des RGB-Bildes in ein HSV-Farbraumbild, und
Verwenden eines Sättigungswertes auf dem HSV-Farbraumbild der auf dem Filter (105) verbleibenden Verunreinigungen als numerischer Wert, der die Menge der auf dem Filter (105) verbleibenden Verunreinigungen darstellt.

3. Automatisches Analyseverfahren nach Anspruch 1, wobei
das automatische Analyseverfahren ferner das Erfassen (S50) eines RGB-Bildes als das Bild umfasst, und
das Schätzen (S51) der Konzentration der Bakterien umfasst:
Umwandeln des RGB-Bildes in ein HSV-Farbraumbild, und
Verwenden eines Merkmalsbetrags, der durch einen Farbtonwert, einen Sättigungswert und einen Helligkeitswert auf dem HSV-Farbraumbild der auf dem Filter (105) verbleibenden Verunreinigungen dargestellt wird, als den numerischen Wert, der die Menge der auf dem Filter (105) verbleibenden Verunreinigungen darstellt.

4. Automatisches Analyseverfahren nach Anspruch 1, wobei das Schätzen (S51) der Konzentration der Bakterien Folgendes umfasst:
Erhalten eines Ergebnisses der Erfassung der Verunreinigungen von einem optischen Sensor (106), der mindestens eine der größten RGB-Farbkomponenten der Verunreinigungen erfasst, und
Verwenden eines Ergebnisses der Erfassung der auf dem Filter (105)
verbliebenen Verunreinigungen durch den optischen Sensor (106) als den numerischen Wert, der die Menge der Verunreinigungen auf dem Filter (105) darstellt.

5. Automatisches Analyseverfahren nach Anspruch 1, wobei das Schätzen (S51) der Konzentration der Bakterien Folgendes umfasst:
Erhalten eines weiteren Detektionsergebnisses durch Detektion der Menge der Verunreinigungen in der Probe, getrennt von der Menge der Verunreinigungen, die bei der Detektion der Menge der auf dem Filter (105) verbliebenen Verunreinigungen detektiert wurde, und
Korrigieren der Menge der Verunreinigungen, die beim Erfassen der Menge der auf dem Filter (105) verbleibenden Verunreinigungen erfasst wurden, unter Verwendung des anderen Erfassungsergebnisses, und Bezugnahme auf die Korrespondenzdaten unter Verwendung der korrigierten Menge der Verunreinigungen.

6. Automatisches Analyseverfahren nach Anspruch 1, wobei
das automatische Analyseverfahren ferner das Durchführen (S54) eines Arzneimittelempfindlichkeitstests der Bakterien gegenüber einem Arzneimittel umfasst, und
das Durchführen (S54) des Arzneimittelempfindlichkeitstests, nachdem die Konzentration der Bakterien in der Probe geschätzt wurde, die Durchführung des Arzneimittelempfindlichkeitstests für die Bakterien in der Probe ohne Kultivierung der Bakterien in der Probe umfasst.

7. Automatisches Analyseverfahren nach Anspruch 6, wobei
das automatische Analyseverfahren ferner das Verdünnen (S52) der Probe umfasst, und
bei dem Verdünnen (S52) der Probe, nachdem die Konzentration der Bakterien in der Probe geschätzt wurde (S51), die Probe verdünnt wird, um eine Testprobe mit einer Konzentration der Bakterien zu erzeugen, die für das Durchführen des Arzneimittelempfindlichkeitstests erforderlich ist, und
das Durchführen (S54) des Arzneimittelempfindlichkeitstests die Durchführung des Arzneimittelempfindlichkeitstests an der durch die Verdünnung der Probe erzeugten Testprobe umfasst.

8. Automatisches Analyseverfahren nach Anspruch 6, wobei das Durchführen (S54) des Drogenempfindlichkeitstests Folgendes umfasst:
Erfassen eines Bildes einer Probe, die in einem auf 35 bis 37°C gehaltenen Inkubator platziert ist, durch eine Abbildungsvorrichtung (106), und
Bestimmen einer minimalen Hemmkonzentration des Arzneimittels durch Messen einer Wachstumsrate der Bakterien unter Verwendung eines Bildes der Probe, das von der Abbildungsvorrichtung (106) aufgenommen wurde.

9. Automatisches Analyseverfahren nach Anspruch 1, wobei die Filtrationsporengröße des Filters (105) 1 bis 40 µm beträgt und ein Material des Filters (105) ein hydrophobes Material ist.

10. Automatisches Analyseverfahren nach Anspruch 1, wobei die Verunreinigungen mindestens rote Blutkörperchen im Blut umfassen.

11. Automatisches Analysegerät (100) zum Analysieren einer Blutprobe, die Bakterien und Verunreinigungen enthält, wobei das automatische Analysegerät (100) umfasst:
einen Zentrifugalseparator (102), der dazu ausgelegt ist, die Verunreinigungen und die Bakterien in der Probe voneinander zu trennen, in die eine Substanz eingebracht wurde, die die Verunreinigungen zerstört;
ein Filtrationsfilter (105), das dazu ausgelegt ist, die Bakterien aus der Probe zu entfernen, von der die Verunreinigungen und die Bakterien durch Filtration der Probe getrennt wurden;
eine Speichereinheit (107), die dazu ausgelegt ist, Korrespondenzdaten zu speichern, die die Korrespondenz zwischen einem numerischen Wert, der eine Menge der auf dem Filter (105) verbleibenden Verunreinigungen darstellt, und einer Konzentration der Bakterien in der Probe beschreiben; und
eine Recheneinheit (110), die dazu ausgelegt ist, eine Konzentration der Bakterien in der Probe durch Bezugnahme auf die Korrespondenzdaten unter Verwendung eines numerischen Wertes zu schätzen, der die Menge der Verunreinigungen darstellt, die auf dem Filter (105) verbleiben, nachdem die Bakterien aus der Probe entfernt wurden,
wobei
die Probe Blutzellen als die Verunreinigungen enthält,
die Substanz ein Tensid ist,
das Tensid mindestens eines der folgenden Elemente umfasst:
ein anionisches Tensid mit einem hydrophilen Teil und einem hydrophoben Teil, wobei der hydrophobe Teil ein Kettenkohlenwasserstoff ist; oder
ein oberflächenaktives Mittel mit einem hydrophilen und einem hydrophoben Teil, wobei der hydrophobe Teil einen zyklischen Kohlenwasserstoff enthält, und
die Recheneinheit (110) dazu ausgelegt ist, die Konzentration der Bakterien in der Probe zu schätzen, indem sie
die Menge der auf dem Filter (105) verbliebenen Verunreinigungen unter Verwendung eines Bildes erfasst, das durch Abbilden ohne Färben der auf dem Filter (105) verbliebenen Verunreinigungen erhalten wird, und
Bezug nimmt auf die Korrespondenzdaten unter Verwendung der Menge der anhand des Bildes erkannten Verunreinigungen.

## Revendications

1. Procédé d'analyse automatique pour analyser un échantillon de sang contenant des bactéries et des impuretés, le procédé comprenant :
l'introduction (S10) d'une substance qui détruit les impuretés dans l'échantillon ;
la séparation (S11) par un séparateur centrifuge (102) des impuretés et des bactéries les unes des autres dans l'échantillon dans lequel la substance a été introduite ;
l'extraction (S12) des bactéries de l'échantillon en utilisant un filtre (105) qui extrait les bactéries de l'échantillon duquel les impuretés et les bactéries ont été séparées ;
la lecture de données de correspondance décrivant une correspondance entre une valeur numérique représentant une quantité des impuretés subsistant sur le filtre (105) et une concentration des bactéries dans l'échantillon depuis une unité (107) de stockage qui stocke les données de correspondance ; et
l'estimation (S51) d'une concentration des bactéries dans l'échantillon en se référant aux données de correspondance en utilisant une valeur numérique représentant la quantité des impuretés subsistant sur le filtre (105) après extraction des bactéries de l'échantillon,
dans lequel
l'échantillon contient des cellules sanguines comme les impuretés,
la substance est un tensio-actif,
le tensio-actif comprend au moins un parmi :
un tensio-actif anionique ayant un groupe caractéristique hydrophile et un groupe caractéristique hydrophobe, le groupe caractéristique hydrophobe étant une chaîne hydrocarbonée ; ou
un tensio-actif ayant un groupe caractéristique hydrophile et un groupe caractéristique hydrophobe, le groupe caractéristique hydrophobe ayant un hydrocarbure cyclique,
le filtre (105) est un filtre de filtration qui sépare les impuretés et les bactéries en filtrant l'échantillon, et
l'estimation (S51) de la concentration des bactéries inclut
la détection de la quantité d'impuretés subsistant sur le filtre (105) en utilisant une image obtenue par imagerie sans coloration des impuretés subsistant sur le filtre (105), et
la référence aux données de correspondance en utilisant la quantité des impuretés détectées en utilisant l'image.

2. Procédé d'analyse automatique selon la revendication 1, dans lequel
le procédé d'analyse automatique comprend en outre la capture (S50) d'une image RVB comme l'image, et
l'estimation (S51) de la concentration des bactéries inclut
la conversion de l'image RVB en une image en espace couleurs HSV, et
l'utilisation d'une valeur de saturation sur l'image en espace couleurs HSV des impuretés subsistant sur le filtre (105) comme la valeur numérique représentant la quantité des impuretés subsistant sur le filtre (105).

3. Procédé d'analyse automatique selon la revendication 1, dans lequel
le procédé d'analyse automatique comprend en outre la capture (S50) d'une image RVB comme l'image, et
l'estimation (S51) de la concentration des bactéries inclut
la conversion de l'image RVB en une image en espace couleurs HSV, et
l'utilisation d'une quantité de caractéristiques représentée par une valeur de teinte, une valeur de saturation, et une valeur de luminosité sur l'image en espace couleurs HSV des impuretés subsistant sur le filtre (105) comme la valeur numérique représentant la quantité des impuretés subsistant sur le filtre (105).

4. Procédé d'analyse automatique selon la revendication 1, dans lequel l'estimation (S51) de la concentration des bactéries inclut
l'obtention d'un résultat de détection des impuretés provenant d'un capteur optique (106) qui détecte au moins la plus grande des composantes couleur RVB des impuretés, et
l'utilisation d'un résultat de détection des impuretés subsistant sur le filtre (105) par le capteur optique (106) comme la valeur numérique représentant la quantité des impuretés subsistant sur le filtre (105).

5. Procédé d'analyse automatique selon la revendication 1, dans lequel l'estimation (S51) de la concentration des bactéries inclut
l'obtention d'un autre résultat de détection en détectant la quantité des impuretés dans l'échantillon, séparément de la quantité des impuretés détectée lors de la détection de la quantité des impuretés subsistant sur le filtre (105), et
la correction de la quantité des impuretés détectée lors de la détection de la quantité des impuretés subsistant sur le filtre (105) en utilisant l'autre résultat de détection, et en faisant référence aux données de correspondance en utilisant la quantité corrigée des impuretés.

6. Procédé d'analyse automatique selon la revendication 1, dans lequel
le procédé d'analyse automatique comprend en outre la réalisation (S54) d'un test de sensibilité aux médicaments des bactéries à un médicament, et
la réalisation (S54) du test de sensibilité aux médicaments inclut, après que la concentration des bactéries dans l'échantillon a été estimée, la réalisation du test de sensibilité aux médicaments des bactéries dans l'échantillon sans mise en culture des bactéries dans l'échantillon.

7. Procédé d'analyse automatique selon la revendication 6, dans lequel
le procédé d'analyse automatique comprend en outre la dilution (S52) de l'échantillon, et
la dilution (S52) de l'échantillon inclut, après que la concentration des bactéries dans l'échantillon a été estimée (S51), la dilution de l'échantillon pour créer un échantillon test ayant une concentration des bactéries nécessaire pour la réalisation du test de sensibilité aux médicaments, et
la réalisation (S54) du test de sensibilité aux médicaments inclut la réalisation du test de sensibilité aux médicaments sur l'échantillon test créé lors de la dilution de l'échantillon.

8. Procédé d'analyse automatique selon la revendication 6, dans lequel la réalisation (S54) du test de sensibilité aux médicaments inclut
la capture d'une image d'un échantillon placé dans un incubateur maintenu à 35 à 37°C par un dispositif (106) d'imagerie, et
la détermination d'une quantité inhibitrice minimum du médicament en mesurant un taux de croissance des bactéries en utilisant une image de l'échantillon capturée par le dispositif (106) d'imagerie.

9. Procédé d'analyse automatique selon la revendication 1, dans lequel une taille de pores de filtration du filtre (105) est 1 à 40 µm, et un matériau du filtre (105) est un matériau hydrophobe.

10. Procédé d'analyse automatique selon la revendication 1, dans lequel les impuretés incluent au moins des globules rouges dans le sang.

11. Analyseur automatique (100) pour analyser un échantillon de sang contenant des bactéries et des impuretés, l'analyseur automatique (100) comprenant :
un séparateur centrifuge (102) qui est adapté à séparer les impuretés et les bactéries les unes des autres dans l'échantillon dans lequel une substance qui détruit les impuretés a été introduite ;
un filtre (105) de filtration qui est adapté à extraire les bactéries de l'échantillon duquel les impuretés et les bactéries ont été séparées en filtrant l'échantillon ;
une unité (107) de stockage qui est adaptée à stocker des données de correspondance décrivant une correspondance entre une valeur numérique représentant une quantité des impuretés subsistant sur le filtre (105) et une concentration des bactéries dans l'échantillon ; et
une unité (110) arithmétique qui est adaptée à estimer une concentration des bactéries dans l'échantillon en se référant aux données de correspondance en utilisant une valeur numérique représentant la quantité des impuretés subsistant sur le filtre (105) après extraction des bactéries de l'échantillon,
dans lequel
l'échantillon contient des cellules sanguines comme les impuretés,
la substance est un tensio-actif,
le tensio-actif comprend au moins un parmi :
un tensio-actif anionique ayant un groupe caractéristique hydrophile et un groupe caractéristique hydrophobe, le groupe caractéristique hydrophobe étant une chaîne hydrocarbonée ; ou
un tensio-actif ayant un groupe caractéristique hydrophile et un groupe caractéristique hydrophobe, le groupe caractéristique hydrophobe ayant un hydrocarbure cyclique, et
l'unité (110) arithmétique est adaptée à estimer la concentration des bactéries dans l'échantillon en
détectant la quantité d'impuretés subsistant sur le filtre (105) en utilisant une image obtenue par imagerie sans coloration des impuretés subsistant sur le filtre (105), et
faisant référence aux données de correspondance en utilisant la quantité des impuretés détectées en utilisant l'image.
